# FASCICULE DE BREVET EUROPEEN

(11) **EP 4 255 825 B1**
(45) Date de publication et mention de la délivrance du brevet: **05.02.2025**
(21) Numéro de dépôt: 21847984.8
(22) Date de dépôt: 02.12.2021
(51) Int. Cl.: B65D 83/64, A61M 15/00, B65D 83/54

(54) **DISPOSITIF DE DISTRIBUTION DE PRODUIT FLUIDE COMPORTANT UN INDICATEUR DE DOSES**
VORRICHTUNG ZUR ABGABE EINES FLÜSSIGPRODUKTS MIT DOSISANZEIGE
FLUID PRODUCT DISPENSING DEVICE COMPRISING A DOSE INDICATOR

(30) Priorité: 04.12.2020 FR 2012715
(43) Date de publication de la demande: 11.10.2023
(73) Titulaire: APTAR France SAS, 27110 Le Neubourg (FR)
(72) Inventeur: SAVALLE, Matthieu, 76350 Oissel (FR)
(74) Mandataire: CAPRI
(86) Numéro de dépôt international: PCT/FR2021/052184
(87) Numéro de publication internationale: WO 2022/117966

(56) Documents cités:
- FR-A1- 3 077 013
- JP-A- 2007 153 415
- JP-U- H0 169 786
- US-A1- 2002 189 611
- US-A1- 2015 157 814

## Description

La présente invention concerne un dispositif de distribution de produit fluide comportant une valve doseuse.

Les valves dite doseuses, dans lesquelles à chaque actionnement de la valve, une dose précise de produit fluide est distribuée, sont bien connues dans l'état de la technique, et sont généralement assemblées sur un réservoir contenant le produit fluide et un gaz propulseur utilisé pour réaliser l'expulsion de la dose.

On connaît principalement deux types de valves doseuses.

Les valves dites à rétention comportent une soupape qui, en position de repos, obture partiellement la chambre de dosage. Plus précisément, l'extérieur de la soupape coopère de manière étanche avec le joint de chambre de la chambre de dosage, de sorte que la chambre de dosage n'est reliée au réservoir, dans cette position de repos, que via le canal interne de la soupape.

Les chambres de dosage des valves dites sans amorçage ne se remplissent que juste avant l'actionnement proprement dit.

Dans les deux cas, le remplissage du réservoir avec le produit fluide à distribuer se fait généralement après assemblage de la valve doseuse sur le réservoir, à travers ladite valve doseuse.

Un élément important pour une valve doseuse consiste à pouvoir indiquer à l'utilisateur l'état de remplissage du réservoir, pour lui permettre d'évaluer si celui-ci sera bientôt vide. Il existe de nombreux indicateurs ou compteurs de doses, très complexes, qui sont compliqués et coûteux à fabriquer et à assembler. Or, dans certains cas, notamment avec certains médicaments, une indication approximative peut être suffisante, et le surcoût important généré par les dispositifs existants n'est pas justifié.

Les documents US2002189611, US2015157814, JPH0169786 et JP2007153415 décrivent des dispositifs de l'état de la technique.

La présente invention a pour but de fournir un dispositif de distribution de produit fluide qui ne reproduit pas les inconvénients susmentionnés.

La présente invention a ainsi pour but de fournir un tel dispositif qui permet d'indiquer à l'utilisateur le niveau de remplissage du réservoir.

La présente invention a également pour but de fournir un tel dispositif qui permet de réutiliser le corps et l'indicateur avec différents distributeurs, quel que soit le niveau de remplissage du réservoir, tout en assurant une indication fiable dudit niveau de remplissage.

La présente invention a aussi pour but de fournir un tel dispositif qui soit simple et peu coûteux à fabriquer et à assembler, et de fonctionnement fiable.

La présente invention a donc pour objet un dispositif de distribution de produit fluide, comportant un corps pourvu d'un manchon axial creux recevant un distributeur de produit fluide comprenant un réservoir contenant un produit fluide et une valve doseuse, ledit produit fluide comprenant une formulation liquide constituée d'un ou plusieurs principe(s) actif(s) en suspension et/ou en solution dans un gaz propulseur liquéfié, ledit corps comportant un indicateur pour indiquer à l'utilisateur la quantité de produit fluide qui reste dans le distributeur de produit fluide, ledit indicateur comportant un élément d'indication disposé à l'extérieur dudit manchon axial creux et déplaçable axialement par rapport audit manchon axial creux le long de moyens d'indication disposés à l'extérieur dudit manchon axial creux, ledit élément d'indication étant déplacé à chaque actionnement du dispositif en l'absence de tout contact mécanique entre ledit élément d'indication et une partie du distributeur mobile durant l'actionnement, tel que ledit réservoir, ledit élément d'indication étant relié par une liaison à une tige interne disposée à l'intérieur dudit manchon axial creux, ladite liaison coulissant dans une rainure axiale réalisée dans ledit manchon axial creux, ladite tige interne formant ou comportant un premier aimant, ledit réservoir contenant un piston suiveur et un ressort de piston qui sollicite ledit piston suiveur axialement en direction de ladite valve doseuse, ledit piston suiveur étant adapté à coulisser axialement dans ledit réservoir de manière étanche après chaque actionnement, pour compenser le volume de chaque dose distribuée, ledit piston suiveur contenant un second aimant, de sorte qu'à chaque déplacement axial dudit piston suiveur après chaque actionnement, celui-ci entraine magnétiquement ladite tige interne et donc ledit élément d'indication, qui se déplace donc axialement le long de ladite rainure axiale.

Avantageusement, ladite valve doseuse comprend un corps de valve contenant une chambre de dosage, une soupape coulissant axialement dans ledit corps de valve entre une position de repos et une position de distribution, pour distribuer le contenu de ladite chambre de dosage, ladite soupape étant sollicitée vers sa position de repos par un ressort de valve.

Avantageusement, ledit gaz propulseur comprend un gaz HFA, tel que le HFA 134a et/ou le HFA 227 et/ou le HFA-152a.

En variante, ledit gaz propulseur comprend le HFO1234ze.

Ces caractéristiques et avantages et d'autres de la présente invention apparaîtront plus clairement au cours de la description détaillée suivante de celle-ci, faite en référence aux dessins joints, donnés à titre d'exemples non limitatifs, et sur lesquels:
La figure 1 est une vue schématique en perspective découpée d'un dispositif de distribution de produit fluide, avant le premier actionnement,
La figure 2 est une vue similaire à celle de la figure 1, après plusieurs actionnements, avec un réservoir presque vide, et
La figure 3 est une vue schématique en perspective, montrant l'indicateur tel que visible de l'extérieur avant le premier actionnement du dispositif.

Dans la description ci-après, les termes "haut", "bas", "supérieur" et "inférieur" se réfèrent à la position droite représentée sur les figures, et les termes "axial" et "radial" se réfèrent à l'axe central longitudinal du dispositif. Les termes "proximal" et "distal" se réfèrent à l'embout de distribution.

Le dispositif représenté sur les figures comporte un corps 100 recevant un distributeur de produit fluide.

Le corps 100 comporte un manchon axial creux 101 recevant le distributeur de produit fluide et un embout de distribution 110, qui dans l'exemple représenté est un embout buccal. Il est toutefois entendu que la présente invention pourrait aussi s'appliquer à un embout de distribution différent, notamment du type nasal. L'embout de distribution 110 définit un orifice de distribution à travers lequel l'utilisateur va inhaler lors de l'utilisation du dispositif. L'embout de distribution 110 peut être réalisé d'une pièce avec le corps 100. Un capot de protection amovible (non représenté) peut être prévu sur ledit embout buccal 110, notamment lors des périodes de stockage, que l'utilisateur va retirer avant utilisation. Le corps 100 peut être réalisé en une seule pièce ou en plusieurs pièces assemblées et fixées les unes aux autres.

Le corps 100 comporte en outre un indicateur 200 pour indiquer à l'utilisateur la quantité de produit fluide qui reste dans le distributeur de produit fluide. Cet indicateur sera plus amplement décrit ci-après.

Le distributeur de produit fluide comporte un réservoir 1 contenant le produit fluide, et une valve doseuse 10. Cette valve doseuse 10 est assemblée sur le réservoir 1, de préférence au moyen d'un élément de fixation 2, qui peut être une capsule à sertir, à visser ou à encliqueter, et avantageusement avec interposition d'un joint de col 3. Éventuellement, une bague 4 peut être assemblée autour du corps 11 de la valve doseuse 10, notamment pour diminuer le volume mort en position inversée et pour limiter le contact du produit fluide avec le joint de col 3. Cette bague 4 peut être de forme quelconque, et l'exemple des figures n'est pas limitatif. Le produit fluide peut être un produit pharmaceutique, cosmétique ou de parfumerie. En particulier, le réservoir 1 peut contenir le produit fluide et le gaz propulseur, en particulier une formulation liquide constituée d'un ou plusieurs principe(s) actif(s) en suspension et/ou en solution dans un gaz propulseur liquéfié, ainsi qu'éventuellement des excipients. Le gaz propulseur comprend avantageusement un gaz HFA, par exemple du HFA 134a et/ou du HFA 227. De préférence le gaz propulseur comprend du HFA-152a. En variante, on peut utiliser d'autres gaz non nocifs pour l'environnement, tel que le HFO1234ze. Le produit fluide contenu dans le réservoir est ainsi pressurisé.

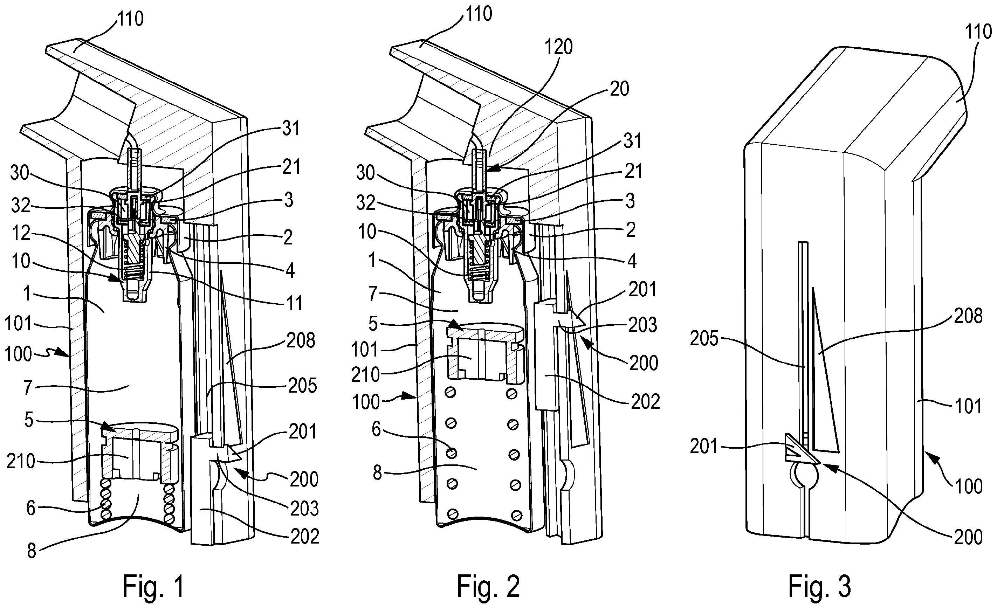

La valve doseuse 10 comporte un corps de valve 11 s'étendant le long d'un axe central longitudinal. À l'intérieur dudit corps de valve 10, une soupape 20 coulisse entre une position de repos, qui est celle représentée sur les figures 1 et 2, et une position de distribution dans laquelle la soupape 20 est enfoncée à l'intérieur du corps de valve 11.

La soupape 20 est sollicitée vers sa position de repos par un ressort de valve 12, qui est disposé dans le corps de valve 11 et qui coopère d'une part avec ce corps de valve 11, et d'autre part avec la soupape 20, de préférence avec une collerette radiale 21 de la soupape 20. Une chambre de dosage 30 est définie à l'intérieur du corps de valve 11, ladite soupape 20 coulissant à l'intérieur de ladite chambre de dosage 30 pour permettre la distribution du contenu de celle-ci lorsque la valve doseuse est actionnée.

De manière connue, la soupape 20 peut être réalisée en deux parties, à savoir une partie haute (également appelée haut de soupape) et une partie basse (également appelée bas de soupape).

De manière classique, lors de l'actionnement, la soupape 20 est fixe par rapport au corps 100, et c'est le réservoir 1 qui est déplacé axialement par rapport au corps 100 entre une position distale, qui est la position de repos, et une position proximale, qui est la position d'actionnement.

L'orifice de sortie de la soupape 20 est relié via un canal audit embout de distribution 110, à travers lequel l'utilisateur inhale le produit distribué. De manière connue, ladite soupape 20 est reçue dans un puits de soupape 120 qui définit au moins partiellement ledit canal.

La chambre de dosage 30 est de préférence définie entre deux joints annulaires, un joint de soupape 31 et un joint de chambre 32, de manière bien connue.

Les figures 1 et 2 représentent la valve doseuse en position droite de stockage, c'est-à-dire la position dans laquelle la chambre de dosage 30 est disposée au-dessus du réservoir 1.

Le réservoir 1 contient un piston suiveur 5 et un ressort de piston 6 qui sollicite le piston suiveur 5 axialement vers le haut en direction de ladite valve doseuse 10. Ainsi, le piston suiveur 5 est soumis d'un côté à la pression exercée sur lui par le produit fluide pressurisé, et de l'autre côté à la pression exercée sur lui par le ressort de piston 6. Le piston suiveur 5 est adapté à coulisser axialement dans le réservoir 1 de manière étanche après chaque actionnement, pour compenser le volume de chaque dose distribuée.

L'insertion du piston suiveur 5 dans le réservoir peut par exemple être réalisée à travers un fond ouvert du réservoir obturé, après insertion, par fixation d'un élément de fermeture approprié. En variante, cette insertion pourrait aussi être réalisée par un haut ouvert du réservoir suivi par la fixation d'une partie de col de réservoir.

Avantageusement, le ressort de piston 6 exerce une faible pression, suffisante pour compenser chaque diminution de produit fluide après chaque actionnement, mais sans exercer une trop forte pression sur le piston suiveur, qui pourrait empêcher ou compliquer le remplissage du réservoir 1. Le choix du ressort de piston 6 et de ses propriétés dépend donc des paramètres du dispositif, tels que les dimensions du réservoir 1, le volume de chaque dose, le type de formulation, la pression du gaz propulseur, etc.

Avant remplissage du réservoir 1 avec le produit fluide, le piston suiveur 5 est donc poussé par le ressort de piston 6 à proximité de la valve doseuse 10, comme visible sur la figure 2.

Lors du remplissage, le produit fluide pousse le piston suiveur 5 axialement vers le bas en comprimant le ressort de piston 6 et l'air disposé entre le piston suiveur 5 et le fond du réservoir 1, jusqu'à ce qu'un équilibre des pressions se fait entre la partie supérieure 7 du réservoir contenant le produit fluide sous forme liquide et la partie inférieure 8 du réservoir contenant l'air et le ressort de piston 6 comprimés. Cette position de fin de remplissage est représentée sur la figure 1.

Après chaque actionnement, suite à la distribution d'une dose de produit fluide hors du réservoir, le volume de la partie supérieure 7 diminue, et le ressort de piston 6 pousse le piston suiveur 5 axialement vers le haut pour compenser cette diminution de volume sans modification substantielle de la pression dans la partie supérieure 7 du réservoir. Ainsi, le produit fluide reste toujours sous forme liquide, ce qui élimine ou pour le moins réduit fortement les risques de "drain back".

Un autre avantage de cette mise en oeuvre est la possibilité d'actionner la valve dans n'importe quelle orientation, car la régulation du volume et de la pression dans la partie supérieure 7 du réservoir par le piston suiveur 5 et son ressort de piston 6 permet non seulement d'empêcher le "drain back" mais aussi d'assurer un bon remplissage de la chambre de dosage 30 après chaque actionnement.

Selon l'invention, l'indicateur 200 comporte un élément d'indication 201 disposé à l'extérieur du manchon axial creux 101 du corps 100, et déplaçable axialement par rapport audit manchon axial creux 101 le long de moyens d'indication 208 disposés à l'extérieur dudit manchon axial creux 101.

Dans l'exemple représenté sur les figures, les moyens d'indication 208 comprennent un triangle coloré se rétrécissant vers le haut, mais tous types de moyens d'indication 208 sont envisageables, par exemple des repères alphanumériques.

Selon l'invention, l'élément d'indication 201 est relié par une liaison 203 à une tige interne 202 disposée à l'intérieur dudit manchon axial creux 101. Ladite liaison 203 coulisse dans une rainure axiale 205 réalisée dans ledit manchon axial creux 101, comme visible sur la figure 3.

Selon l'invention, l'élément d'indication 201 est déplacé à chaque actionnement du dispositif en l'absence de tout contact mécanique entre ledit élément d'indication 201 et une partie du dispositif mobile durant l'actionnement, comme par exemple le réservoir 1 ou l'élément de fixation 2. L'indicateur 200 est donc totalement indépendant de la valve doseuse 10. De plus, le corps 100 et l'indicateur 200 sont aisément réutilisables en remplaçant le distributeur de produit fluide.

Selon l'invention, ladite tige interne 202 forme ou comporte un premier aimant. Parallèlement, le piston suiveur 5 contient un second aimant 210. Ainsi, à chaque déplacement axial du piston suiveur 5 après chaque actionnement, celui-ci entraine magnétiquement la tige interne 202 et donc l'élément d'indication 201, qui se déplace donc axialement le long de la rainure axiale 205.

Le choix des premier et second aimants est réalisée pour d'une part garantir un actionnement fiable de l'indicateur 200 sans d'autre part générer trop de frottements qui pourraient impacter le bon fonctionnement du dispositif et/ou de l'indicateur. Les propriétés de ces aimants (dimensions, poids, force, etc.) sont ajustées en fonction des spécificités, notamment des dimensions, du dispositif.

Un avantage particulier d'un élément d'indication 201 qui suit magnétiquement un piston suiveur 5 dans le réservoir est qu'en cas de remplacement du distributeur, l'élément d'indication 201 vient automatiquement se positionner en regard du piston suiveur 5, avec une indication fiable concernant le remplissage du réservoir. Ceci permet de réutiliser le corps 100 et son indicateur 200 avec différents distributeurs, quel que soit le niveau de remplissage du réservoir, sans risquer de fournir une indication erronée sur ce niveau de remplissage.

On pourrait envisager différentes solutions alternatives pour déplacer sans contact l'élément d'indication 201 à chaque actionnement, par exemple un flotteur pourvu d'un aimant. En variante, on pourrait prévoir de disposer à l'intérieur du corps 100 un électro-aimant activé à chaque actionnement, par exemple par un contacteur déclenché par le réservoir 1 ou l'élément de fixation 2 en position d'actionnement, cet électro-aimant lorsqu'activé agissant sur une partie interne de l'élément d'indication pour le déplacer d'une courte distance à chaque actionnement. D'autres variantes sont aussi possibles.

Bien que la présente invention ait été décrite en référence à un mode de réalisation particulier de celle-ci, il est entendu qu'elle n'est pas limitée par l'exemple représenté. Au contraire, l'homme du métier peut y apporter toutes modifications utiles sans sortir du cadre de la présente invention tel que défini par les revendications annexées.

## Revendications

1. Dispositif de distribution de produit fluide, comportant un corps (100) pourvu d'un manchon axial creux (101) recevant un distributeur de produit fluide comprenant un réservoir (1) contenant un produit fluide et une valve doseuse (10), ledit produit fluide comprenant une formulation liquide constituée d'un ou plusieurs principe(s) actif(s) en suspension et/ou en solution dans un gaz propulseur liquéfié, ledit corps (100) comportant un indicateur (200) pour indiquer à l'utilisateur la quantité de produit fluide qui reste dans le distributeur de produit fluide, ledit indicateur (200) comportant un élément d'indication (201) disposé à l'extérieur dudit manchon axial creux (101) et déplaçable axialement par rapport audit manchon axial creux (101) le long de moyens d'indication (208) disposés à l'extérieur dudit manchon axial creux (101), ledit élément d'indication (201) étant déplacé à chaque actionnement du dispositif en l'absence de tout contact mécanique entre ledit élément d'indication (201) et une partie du distributeur mobile durant l'actionnement, tel que ledit réservoir (1), ledit élément d'indication (201) étant relié par une liaison (203) à une tige interne (202) disposée à l'intérieur dudit manchon axial creux (101), ladite liaison (203) coulissant dans une rainure axiale (205) réalisée dans ledit manchon axial creux (101), ladite tige interne (202) formant ou comportant un premier aimant, ledit réservoir (1) contenant un piston suiveur (5) et un ressort de piston (6) qui sollicite ledit piston suiveur (5) axialement en direction de ladite valve doseuse (10), ledit piston suiveur (5) étant adapté à coulisser axialement dans ledit réservoir (1) de manière étanche après chaque actionnement, pour compenser le volume de chaque dose distribuée, ledit piston suiveur (5) contenant un second aimant (210), de sorte qu'à chaque déplacement axial dudit piston suiveur (5) après chaque actionnement, celui-ci entraine magnétiquement ladite tige interne (202) et donc ledit élément d'indication (201), qui se déplace donc axialement le long de ladite rainure axiale (205).

2. Dispositif selon la revendication 1, dans lequel ladite valve doseuse (10) comprend un corps de valve (11) contenant une chambre de dosage (30), une soupape (20) coulissant axialement dans ledit corps de valve (11) entre une position de repos et une position de distribution, pour distribuer le contenu de ladite chambre de dosage (30), ladite soupape (20) étant sollicitée vers sa position de repos par un ressort de valve (12).

3. Dispositif selon la revendication 1 ou 2, dans lequel ledit gaz propulseur comprend un gaz HFA, tel que le HFA 134a et/ou le HFA 227 et/ou le HFA-152a.

4. Dispositif selon la revendication 1 ou 2, dans lequel ledit gaz propulseur comprend le HFO1234ze.

## Patentansprüche

1. Vorrichtung zur Abgabe eines Fluidprodukts, umfassend einen Körper (100), der mit einer axial verlaufenden Hohlhülse (101) versehen ist, die einen Fluidproduktspender aufnimmt, der ein Reservoir (1) mit einem Fluidprodukt und ein Dosierventil (10) umfasst, wobei das Fluidprodukt eine flüssige Formulierung enthält, die aus einem oder mehreren Wirkstoff(en) in Suspension und/oder in Lösung in einem verflüssigten Treibgas besteht, wobei der Körper (100) einen Indikator (200) aufweist, um dem Benutzer die Menge an Fluidprodukt anzuzeigen, die in dem Fluidproduktspender verbleibt, wobei der Indikator (200) ein Anzeigeelement (201) aufweist, das außerhalb der axial verlaufenden Hohlhülse (101) angeordnet ist und axial bezüglich der axial verlaufenden Hohlhülse (101) entlang von Anzeigemitteln (208), die außerhalb der axial verlaufenden Hohlhülse (101) angeordnet sind, verschiebbar ist, wobei das Anzeigeelement (201) bei jeder Betätigung der Vorrichtung unter Ausbleiben von jeglichem mechanischen Kontakt zwischen dem Anzeigeelement (201) und einem Teil des Spenders, das während der Betätigung beweglich ist, wie etwa dem Reservoir (1), verschoben wird, wobei das Anzeigeelement (201) über eine Verbindung (203) mit einem inneren Stab (202) verbunden ist, der innerhalb der axial verlaufenden Hohlhülse (101) angeordnet ist, wobei die Verbindung (203) in einer axial verlaufenden Nut (205) gleitet, die in der axial verlaufenden Hohlhülse (101) ausgebildet ist, wobei der innere Stab (202) einen ersten Magneten bildet oder aufweist, wobei das Reservoir (1) einen Nachlaufkolben (5) und eine Kolbenfeder (6) enthält, die den Nachlaufkolben (5) axial in Richtung des Dosierventils (10) beaufschlagt, wobei der Nachlaufkolben (5) dazu geeignet ist, nach jeder Betätigung abgedichtet axial in dem Reservoir (1) zu gleiten, um jeweils das Volumen einer abgegebenen Dosis zu kompensieren, wobei der Nachlaufkolben (5) einen zweiten Magneten (210) enthält, so dass jeweils bei einer axialen Verschiebung des Nachlaufkolbens (5) nach jeder Betätigung dieser den inneren Stab (202) und damit das Anzeigeelement (201) magnetisch mitnimmt, das sich somit axial entlang der axial verlaufenden Nut (205) bewegt.

2. Vorrichtung nach Anspruch 1,
wobei das Dosierventil (10) einen Ventilkörper (11) mit einer Dosierkammer (30) und ein Ventilelement (20) aufweist, das in dem Ventilkörper (11) zwischen einer Ruheposition und einer Abgabeposition axial verschiebbar ist, um den Inhalt der Dosierkammer (30) abzugeben, wobei das Ventilelement (20) durch eine Ventilfeder (12) in seine Ruheposition beaufschlagt wird.

3. Vorrichtung nach Anspruch 1 oder 2,
wobei das Treibgas ein HFA-Gas, wie HFA 134a und/oder HFA 227 und/oder HFA-152a, enthält.

4. Vorrichtung nach Anspruch 1 oder 2,
wobei das Treibgas HFO1234ze enthält.

## Claims

1. A fluid product dispensing device, including a body (100) provided with a hollow axial sleeve (101) receiving a fluid product dispenser comprising a reservoir (1) containing a fluid product and a metering valve (10), said fluid product comprising a liquid formulation consisting of one or more active ingredient(s) in suspension and/or in solution in a liquefied propellant gas, said body (100) including an indicator (200) to indicate to the user the amount of fluid product remaining in the fluid product dispenser, said indicator (200) including an indication element (201) disposed outside said hollow axial sleeve (101) and axially movable relative to said hollow axial sleeve (101) along indication means (208) disposed outside said hollow axial sleeve (101), said indication element (201) being moved each time the device is actuated in the absence of any mechanical contact between said indication element (201) and a part of the dispenser that is movable during the actuation, such as said reservoir (1), said indication element (201) being connected by a link (203) to an inner rod (202) disposed inside said hollow axial sleeve (101), said link (203) sliding in an axial groove (205) made in said hollow axial sleeve (101), said inner rod (202) forming or including a first magnet, said reservoir (1) containing a follower piston (5) and a piston spring (6) which biases said follower piston (5) axially towards said metering valve (10), said follower piston (5) being adapted to slide axially in said reservoir (1) in a sealed manner after each actuation, to compensate for the volume of each dispensed dose, said follower piston (5) containing a second magnet (210), so that at each axial displacement of said follower piston (5) after each actuation, the latter magnetically drives said inner rod (202) and therefore said indication element (201), which therefore axially moves along said axial groove (205).

2. The device according to claim 1, wherein said metering valve (10) comprises a valve body (11) containing a metering chamber (30), a valve (20) axially sliding in said valve body (11) between a rest position and a dispensing position, for dispensing the contents of said metering chamber (30), said valve (20) being biased towards its rest position by a valve spring (12).

3. The device according to claim 1 or 2, wherein said propellant gas comprises an HFA gas, such as HFA 134a and/or HFA 227 and/or HFA-152a.

4. The device according to claim 1 or 2, wherein said propellant gas comprises HF01234ze.
